(19) Europäisches Patentamt
European Patent Office
Office européen des brevets

(11) Veröffentlichungsnummer: **0 455 155 B1**

## (12) EUROPÄISCHE PATENTSCHRIFT

(45) Veröffentlichungstag der Patentschrift: **12.07.95**

(21) Anmeldenummer: **91106774.2**

(22) Anmeldetag: **26.04.91**

(51) Int. Cl.6: **C07C 255/59**, A61K 31/275, A23K 1/16

Die Akte enthält technische Angaben, die nach dem Eingang der Anmeldung eingereicht wurden und die nicht in dieser Patentschrift enthalten sind.

(54) **Enantiomerentrennung von Cimaterol.**

(30) Priorität: **04.05.90 DE 4014252**

(43) Veröffentlichungstag der Anmeldung:
**06.11.91 Patentblatt 91/45**

(45) Bekanntmachung des Hinweises auf die Patenterteilung:
**12.07.95 Patentblatt 95/28**

(84) Benannte Vertragsstaaten:
**AT BE CH DE DK ES FR GB GR IT LI LU NL SE**

(56) Entgegenhaltungen:
**GB-A- 1 187 546**
**US-A- 4 119 710**
**US-A- 4 407 819**

(73) Patentinhaber: **BOEHRINGER INGELHEIM VET-MEDICA GMBH**
**Postfach 2 00**
**D-55216 Ingelheim (DE)**

(72) Erfinder: **Resemann, Wolfgang, Dr. Dipl.-Chem.**
**Rosenstrasse 25**
**W-7950 Biberach 1 (DE)**
Erfinder: **Dürr, Adolf,**
**Mozartstrasse 1**
**W-7950 Biberach 1 (DE)**
Erfinder: **Engelhardt, Günther, Prof. Dr.**
**Unterer Bühl 18**
**W-7950 Biberach 1 (DE)**
Erfinder: **Ouirke, John Francis, Dr.**
**Metzrothstrasse 2**
**W-6530 Bingerbrück (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

In der US-A-4.119.710 wird u. a. das Racemat 1-(4'-Amino-3'-cyano-phenyl)-2-isopropylamino-ethanol (generic name: Cimaterol) und dessen pharmazeutische Eigenschaften beschrieben. So weisen die in der US-A-4.119.710 beschriebenen Verbindungen neben einer analgetischen, uterusspasmolytischen und einer antispastischen Wirkung auf die quergestreifte Muskulatur, insbesondere $\beta_2$-mimetische und/oder $\beta_1$-blockierende Wirkungen auf, wobei je nach ihrer Substitution die eine oder andere Wirkung im Vordergrund steht. Außerdem wird in der US-A-4.119.710 festgestellt, daß die d-(+)-Verbindungen insbesondere eine selektive Wirkung auf die $\beta_1$-Rezeptoren und die l-(-)-Verbindungen eine bevorzugte Wirkung auf die $\beta_2$-Rezeptoren aufweisen.

Desweiteren wird in der US-A-4.407.819 u. a. die leistungsfördernde Wirkung von Cimaterol bei Tieren beschrieben.

Es ist ferner bekannt, daß normalerweise eines der beiden Enantiomeren eines Racemats eine größere Wirksamkeit als das andere Enantiomere aufweist. Aus diesem Grunde lag der vorliegenden Anmeldung die Aufgabe zugrunde, das literaturbekannte Racemat in seine neuen Enantiomeren aufzutrennen.

Bei der Lösung dieser Aufgabe traten überraschenderweise Schwierigkeiten auf, obwohl bereits in der US-A-4.119.710 zwei Verfahren zur Racemattrennung dieser Verbindungen beschrieben werden, nämlich

a) die Auftrennung eine Gemisches von diastereomeren Verbindungen, die durch Umsetzung eines entsprechenden Racemats mit einem chiralen Acylrest erhalten werden, und anschließende Abspaltung des chiralen Acylrestes, z.B. des (-)-Menthylcarbonylrestes, oder

b) die fraktionierte Kristallisation eines Gemisches ihrer diastereomeren Salze mit einer optisch aktiven Hilfssäure.

Die Racemattrennung a) erschien dabei von Anfang an wegen der im Cimaterol vorhandenen Cyanogruppe wenig erfolgsversprechend zu sein, da bei der anschließend erforderlichen hydrolytischen oder hydrogenolytischen Abspaltung des chiralen Acylrestes die vorhandene Cyanogruppe zumindest teilweise hydrolytisch oder hydrogenolytisch verändert wird.

Die übliche Racemattrennung b), welche darin besteht, daß ein entsprechendes diastereomeres Salz durch Erhitzen in einem geeigneten Lösungsmittel gelöst und anschießend beim Abkühlen das schwerer lösliche diastereomere Salz zuerst auskristallisiert, wobei dieser Vorgang bis zur Isolierung des reinen schwerer löslichen diastereomeren Salzes erforderlichenfalls mehrmals wiederholt werden kann, und anschließend aus dem so erhaltenen reinen diastereomeren Salz die gewünschte enantiomere Base freigesetzt wird, führte überraschenderweise bei Cimaterol nicht zum Ziele, wie aus dem beigefügten Referenzbeispiel hervorgeht.

Überraschenderweise wurde nun gefunden, daß sich racemisches Cimaterol, das bereits eines der gewünschten Enantiomere in angereicherter Form enthalten kann, durch Lösen von Cimaterol und mindestens 2 Äquivalenten, zweckmäßigerweise von 2 bis 7 Äquivalenten, vorzugsweise jedoch von 2 bis 4 Äquivalenten, einer optisch aktiven zweibasischen Hilfssäure wie (-)-0,0'-Dibenzoyl-L-weinsäure oder (+)-0,0'-Dibenzoyl-D-weinsäure unter Einhaltung eines bestimmten Temperaturbereichs, welcher zweckmäßigerweise oberhalb von 20 °C, vorzugsweise jedoch zwischen 25 und 30 °C, liegt, in Methanol über seine beiden diastereomeren Salze und anschließende Freisetzung der enantiomeren Base auftrennen läßt. Hierbei fällt das gewünschte diastereomere Salz mit einer Reinheit von mindestens ee = 90 % nach kurzer Zeit aus, z. B. nach 1 bis 5 Minuten, wobei allerdings der charakteristische Temperaturbereich, welcher durch übliche Handversuche ermittelt werden kann, nicht unterschritten werden darf. Die Lösung der beiden Komponenten erfolgt hierzu vorzugsweise gleichzeitig, eine Lösung der einen Komponenten kann selbstverständlich auch zu einer Lösung der anderen Komponenten gegeben werden.

Besonders vorteilhaft wird die Racemattrennung mit 2 bis 7 Äquivalenten, vorzugsweise mit 1 bis 3 Äquivalenten von (-)-0,0'-Dibenzoyl-L-weinsäure oder (+)-0,0'-Dibenzoyl-D-weinsäure als Hilfssäure bei Temperaturen oberhalb von 25 °C, vorzugsweise jedoch in einem Temperaturbereich zwischen 25 und 30 °C, durchgeführt. So erhält man beispielsweise nach gleichzeitigem Lösen von 1 Mol Cimaterol und 1 Mol (-)-0,0'-Dibenzoyl-L-weinsäure oder (+)-0,0'-Dibenzoyl-D-weinsäure in Methanol jeweils reines (+)-1-(4'-Amino-3'-cyano-phenyl)-2-isopropylamino-ethanol-(-)-0,0'-dibenzoyl-L-hydrogentartrat oder (-)-1-(4'-Amino-3'-cyano-phenyl)-2-isopropylamino-ethanol-(+)-0,0'-dibenzoyl-D-hydrogentartrat, wobei nach kurzer Zeit, z. B. nach 1 bis 2 Minuten, jeweils das diesbezügliche reine diastereomere Hydrogentartrat zu kristallisieren beginnt. Dieses Salz fällt schon nach dem ersten Schritt in einer hohen Reinheit von mindestens ee = 94 % an. Sollte eine noch größere Reinheit erforderlich sein, so kann durch Lösen des nach einmaliger Fällung erhaltenen diastereomeren Hydrogentartrats und erneutes Kristallisieren oberhalb von 25 °C das Trennverfahren einmal oder erforderlichenfalls mehrmals wiederholt werden.

Von dem so erhaltenen diastereomeren Salz wird anschließend mittels einer Base, vorzugsweise mittels verdünntem Ammoniak, die gewünschte reine enantiomere Base freigesetzt und nach üblichen Methoden isoliert.

Die erhaltenen enantiomeren Basen können gewünschtenfalls anschließend in ihre Säureadditionssalze, insbesondere für die pharmazeutische Anwendung in ihre physiologisch verträglichen Salze mit anorganischen oder organischen Säuren, vorzugsweise jedoch mit organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Besonders bemerkenswert bei dem erfindungsgemäßen Verfahren ist jedoch, daß bei einer versehentlichen Unterschreitung des Temperaturbereiches das beispielsweise erhaltene racemische Cimaterol-0,0'-dibenzoyl-L- oder Cimaterol-0,0'-dibenzoyl-D-hydrogentartrat nach erneutem Aufschlämmen und Erwärmen über 25°C, in das gewünschte reine diastereomere Hydrogentartrat übergeführt werden kann.

Ein weiterer Gegenstand der Erfindung ist somit ein Verfahren zur Herstellung der reinen diastereomeren Salze von Cimaterol mit (-)-0,0'-Dibenzoyl-L-weinsäure oder (+)-0,0'-Dibenzoyl-D-weinsäure.

Die nachfolgenden Beispiele sollen die Erfindung näher erläutern:

Referenzbeispiel

(-)-1-(4'-Amino-3'-cyano-phenyl)-2-isopropylamino-ethanol

a) (-)-1-(4'-Amino-3'-cyano-phenyl)-2-isopropylamino-ethanol-(+)-0,0'-dibenzoyl-D-hydrogentartrat
50,0 g (0,23 Mol) Cimaterol und 82,4 g (0,23 Mol) (+)-0,0'-Dibenzoyl-D-weinsäure werden in 500 ml Methanol bei Raumtemperatur gelöst. Nach kurzer Zeit kristallisiert das Hydrogentartrat des Cimaterols aus. Die Kristallsuspension wird ca. 2 Stunden bei 19-20°C Innentemperatur gerührt, abgesaugt und der Filterrückstand mit kaltem Methanol nachgewaschen sowie bei 50°C bis zur Gewichtskonstanz getrocknet.
Ausbeute: 90 g (68,0 % der Theorie bezogen auf eingesetztes Racemat),
Schmelzpunkt der freigesetzten Base: 162,8°C
ee = 8
b) 88 g des gemäß a) erhaltenen Hydrogentartrats werden in 500 ml Methanol bis zur vollständigen Lösung unter Rückfluß gekocht und die klare Lösunge wieder auf ca. 15°C Kolbeninnentemperatur abgekühlt. Das ausfallende Kristallisat wird noch ca. 2-3 Stunden bei 15-18°C gerührt, abgesaugt, mit kaltem Methanol gewaschen und getrocknet.
Ausbeute: 62 g (70,5 % des Einsatzes),
Schmelzpunkt der freigesetzten Base: 160,9°C
ee = 24
c) 60 g des gemäß b) erhaltenen Hydrogentartrats werden in 350 ml Methanol analog a) in der Siedehitze gelöst, das Kristallisat ca. 12 Stunden bei 10-15°C gerührt, abgesaugt, mit kaltem Methanol gewaschen und getrocknet.
Ausbeute: 34 g (56,7 % des Einsatzes),
Schmelzpunkt der freigesetzten Base: 151,6°C
ee = 45
Nach der 2. Umkristallisation betrug die Gesamtausbeute nur noch 27,2 % der Theorie bezogen auf das eingesetzte Racemat, wobei nur ein enantimomerer Überschuß (ee) von 45 erzielt wurde. Wegen der großen Verluste wurde auf eine Fortführung der Kristallisation verzichtet.

Beispiel 1

(-)-1-(4'-Amino-3'-cyano-phenyl)-2-isopropylamino-ethanol

a) (-)-1-(4'-Amino-3'-cyano-phenyl)-2-isopropylamino-ethanol-(+)-0,0'-dibenzoyl-D-hydrogentartrat
50 g (0,23 Mol) 1-(4'-Amino-3'-cyano-phenyl)-2-isopropylamino-ethanolund 82,4 g (0,23 Mol) (+)-0,0'-Dibenzoyl-D-weinsäure werden in einem 1 l-Dreihalskolben in 500 ml Methanol bei 25 bis 28°C unter Rühren gelöst. Aus der klaren Lösung kristallisiert nach ca. 1 bis 2 Minuten das linksdrehende diastereomere Hydrogentartrat. Nach ca. 2 Stunden Nachrührzeit bei 25 bis 28°C wird das Kristallisat abgesaugt, mit wenig Methanol nachgewaschen und bei 50°C im Umlufttrockenschank getrocknet.
Ausbeute: 44 g (66,5 % der Theorie),
Schmelzpunkt: 140-141°C
b) 42 g des gemäß Beispiel 1a erhaltenen Hydrogentartrats werden in einem 1 l Dreihalskolben in der Siedehitze unter Rühren in 550 ml Methanol gelöst und die heiße Lösung in einer 2 l Filterpresse über ein Seitz-EK-Filter klarfiltriert. Das Filtrat wird unter schwachem Vakuum auf ca. 100 - 150 ml Lösung eingeengt und die entstandene Kristallsuspension ca. 1 Stunde bei 25 bis 28°C nachgerührt. Das Kristallisat wird abgesaugt, mit wenig Methanol nachgewaschen und bei 50°C im Umlufttrockenschrank getrocknet.
Ausbeute: 35,7 g (85 % bezogen auf das eingesetzte Hydrogentartrat),
Schmelzpunkt: 140-141°C
c) (-)-1-(4'-Amino-3'-cyano-phenyl)-2-isopropylamino-ethanol

36,0 g des gemäß Beispiel 1b erhaltenen Hydrogentartrats werden bei Raumtemperatur in 50 ml konzentriertem Ammoniak und 150 ml Wasser nach und nach eingetragen. Nach 1 Stunde Rühren wird die Kristallsuspension abfiltriert, gut mit deionisiertem Wasser nachgewaschen und bei Raumtemperatur im Umlufttrockenschrank bis zur Gewichtskonstanz getrocknet.
Ausbeute: 11,7 g (85,6 % der Theorie),
Schmelzpunkt: 146,5 °C
$\alpha$ = - 4,37 °
$[\alpha]_D^{20}$ = - 31 °
ee = 99,4

## Beispiel 2

( + )-1-(4'-Amino-3'-cyano-phenyl)-2-
isopropylamino-ethanol

a) ( + )-1-(4'-Amino-3'-cyano-phenyl)-2-isopropylamino-ethanol-(-)-0,0'-dibenzoyl-L-hydrogentartrat
Hergestellt aus 1-(4'-Amino-3'-cyano-phenyl)-2-isopropylamino-ethanolund (-)-0,0'-Dibenzoyl-L-weinsäure analog Beispiel 1a.
Ausbeute: 69,8 % der Theorie,
Schmelzpunkt: 140-141 °C
b) Die weitere Reinigung erfolgt analog Beipiel 1b. Ausbeute: 83 % bezogen auf das eingesetzte Hydrogentartrat, Schmelzpunkt: 140-141 °C
c) ( + )-1-(4'-Amino-3'-cyano-phenyl)-2-isopropylamino-ethanol
Hergestellt analog Beispiel 1c.
Ausbeute: 81 % der Theorie,
Schmelzpunkt: 146,5 °C
$\alpha$ = + 4,38 °
$[\alpha]_{D'}^{20}$ = + 31,5 °, ee = 98,2

## Beispiel 3

( + )-1-(4'-Amino-3'-cyano-phenyl)-2-
isopropylamino-ethanol

Die aus der Fällung gemäß Beispiel 1a erhaltene Mutterlauge wird im Vakuum zur Trockene eingedampft und der erhaltene Rückstand mit 50 ml konzentriertem Ammoniak und 150 ml Wasser aufgenommen und bei ca. 5 bis 10 °C 2 Stunden lang gerührt. Nach dem Absaugen wird der Filterrückstand mit Wasser gut gewaschen und im Umlufttrockenschrank bei Raumtemperatur gut getrocknet.
Ausbeute: 26,5 g (53 % bezogen auf eingesetztes Racemat)
Das so erhaltene rohe ( + )-1-(4'-Amino-3'-cyano-phenyl)-2-isopropylamino-ethanol wird mit (-)-0,0'-Dibenzoyl-L-weinsäure analog Beispiel 2 gereinigt:

a) Ausbeute: 39,7 g (56,8 % bezogen auf eingesetztes Racemat-Enantiomeren-Gemisch), Schmelzpunkt: 140-141 °C
b) Ausbeute: 87 % bezogen auf das eingesetzte Hydrogentartrat, Schmelzpunkt: 140-141 °C
c) Ausbeute: 78,5 % der Theorie, Schmelzpunkt: 146,5 °C
$\alpha$ = + 4,37 °
$[\alpha]_D^{20}$ = + 31 °, ee = 98

## Beispiel 4

(-)-1-(4'-Amino-3'-cyano-phenyl)-2-isopropylamino-
ethanol

50 g (-)-1-(4'-Amino-3'-cyano-phenyl)-2-isopropylamino-ethanol-( + )-0,0'-dibenzoyl-L-hydrogentartrat (hergestellt gemäß Referenzbeispiel a), Schmelzpunkt der freigesetzten Base: 162,8 °C; ee = 8) werden mit 500 ml Methanol aufgeschlämmt und bei ca. 28 °C Kolbeninnentemperatur eine Stunde gerührt. Anschließend wird abgesaugt, mit wenig Methanol gewaschen und bis zur Gewichtskonstanz bei 50 °C getrocknet. Ausbeute: 28,0 g (56 % bezogen auf Racemat-Enantiomeren-Gemisch).
Das so erhaltene Hydrogentartrat wird analog Beispiel 1c) mittels verdünntem Ammoniak freigesetzt.
Ausbeute: 9,14 g (86 % der Theorie),
Schmelzpunkt: 145 °C
$\alpha$ = - 4,37 °
$[\alpha]_{D'}^{20}$ = - 31 °
ee = 98

## Patentansprüche

1. Verfahren zur Herstellung von (-)-1-(4'-Amino-3'-cyano-phenyl)-2-isopropylamino-ethanol und von ( + )-1-(4'-Amino-3'-cyano-phenyl)-2-isopropylamino-ethanol, dadurch gekennzeichnet, daß 1-(4'-Amino-3'-cyano-phenyl)-2-isopropylamino-ethanol durch Lösen von 1-(4'-Amino-3'-cyano-phenyl)-2-isopropylamino-ethanol und mindestens 2 Äquivalenten (-)-0,0'-Dibenzoyl-L-weinsäure oder ( + )-0,0'-Dibenzoyl-D-weinsäure in Methanol bei Temperaturen oberhalb von 20 °C kristallisiert wird, das kristallisierte diastereomere Salz abgetrennt wird, anschließend die enantiomere Base freigesetzt wird und gewünschtenfalls anschließend die so erhaltene enantiomere Base in ihre physiologisch verträglichen Säureadditionssalze übergeführt wird.

2. Verfahren zur Herstellung der diastereomeren Salze von 1-(4'-Amino-3'-cyano-phenyl)-2-isopropylamino-ethanol mit (-)-0,0'-Dibenzoyl-L-weinsäure oder (+)-0,0'-Dibenzoyl-D-weinsäure, dadurch gekennzeichnet, daß 1-(4'-Amino-3'-cyano-phenyl)-2-isopropylamino-ethanol durch Lösen von 1-(4'-Amino-3'-cyano-phenyl)-2-isopropylamino-ethanol und mindestens 2 Äquivalenten (-)-0,0'-Dibenzoyl-L-weinsäure oder (+)-0,0'-Dibenzoyl-D-weinsäure in Methanol bei Temperaturen oberhalb von 20°C kristallisiert und das kristallisierte diastereomere Salz abgetrennt wird.

3. Verfahren gemäß Anspruch 1 oder 2, dadurch gekennzeichnet, daß die Kristallisation oberhalb von 25°C durchgeführt wird.

4. Verfahren gemäß Anspruch 1 oder 2, dadurch gekennzeichnet, daß die Kristallisation bei Temperaturen zwischen 25 und 30°C durchgeführt wird.

**Claims**

1. Process for preparing (-)-1-(4'-amino-3'-cyano-phenyl)-2-isopropylamino-ethanol and (+)-1-(4'-amino-3'-cyano-phenyl)-2-isopropylamino-ethanol, characterised in that 1-(4'-amino-3'-cyano-phenyl)-2-isopropylamino-ethanol is crystallised by dissolving 1-(4'-amino-3'-cyano-phenyl)-2-isopropylamino-ethanol and at least 2 equivalents of (-)-0,0'-dibenzoyl-L-tartaric acid or (+)-0,0'-dibenzoyl-D-tartaric acid in methanol at temperatures above 20°C, the crystallised diastereomeric salt is separated off, then the enantiomeric base is liberated and subsequently, if desired, the enantiomeric base thus obtained is converted into a physiologically acceptable acid addition salt thereof.

2. Process for preparing the diastereomeric salts of 1-(4'-amino-3'-cyanophenyl)-2-isopropylamino-ethanol with (-)-0,0'-dibenzoyl-L-tartaric acid or (+)-0,0'-dibenzoyl-D-tartaric acid, characterised in that 1-(4'-amino-3'-cyano-phenyl)-2-isopropylamino-ethanol is crystallised by dissolving 1-(4'-amino-3'-cyano-phenyl)-2-isopropylamino-ethanol and at least 2 equivalents of (-)-0,0'-dibenzoyl-L-tartaric acid or (+)-0,0'-dibenzoyl-D-tartaricacid in methanol at temperatures above 20°C and the crystallised diastereomeric salt is separated off.

3. Process according to claim 1 or 2, characterised in that the crystallisation is carried out at above 25°C.

4. Process according to claim 1 or 2, characterised in that the crystallisation is carried out at temperatures between 25 and 30°C.

**Revendications**

1. Procédé de préparation du (-)-1-(4'-amino-3'-cyano-phényl)-2-isopropylamino-éthanol et du (+)-1-(4'-amino-3'-cyano-phényl)-2-isopropylamino-éthanol, caractérisé en ce que le 1-(4'-amino-3'-cyano-phényl)-2-isopropylamino-éthanol est cristallisé par dissolution de 1-(4'-amino-3'-cyano-phényl)-2-isopropylamino-éthanol et d'au moins deux équivalents d'acide (-)-0,0'-dibenzoyl-L-tartrique ou d'acide (+)-0,0'-dibenzoyl-D-tartrique dans le méthanol à des températures supérieures à 20°C, le sel diastéréoisomère cristallisé est séparé, puis la base énantiomère est libérée et, si on le souhaite, la base énantiomère ainsi obtenue est ensuite convertie en ses sels d'addition d'acide physiologiquement acceptables.

2. Procédé de préparation des sels diastéréoisomères du 1-(4'-amino-3'-cyano-phényl)-2-isopropylamino-éthanol avec l'acide (-)-0,0'-dibenzoyl-L-tartrique ou l'acide (+)-0,0'-dibenzoyl-D-tartrique, caractérisé en ce que le 1-(4'-amino-3'-cyano-phényl)-2-isopropylamino-éthanol est cristallisé par dissolution de 1-(4'-amino-3'-cyano-phényl)-2-isopropylamino-éthanol et d'au moins deux équivalents d'acide (-)-0,0'-dibenzoyl-L-tartrique ou d'acide (+)-0,0'-dibenzoyl-D-tartrique dans le méthanol à des températures supérieures à 20°C et le sel diastéréoisomère cristallisé est séparé.

3. Procédé selon la revendication 1 ou 2, caractérisé en ce que la cristallisation est réalisée à plus de 25°C.

4. Procédé selon la revendication 1 ou 2, caractérisé en ce que la cristallisation est réalisée à des températures situées entre 25 et 30°C.